# EUROPÄISCHE PATENTSCHRIFT

(11) **EP 2 060 226 B1**
(45) Veröffentlichungstag und Bekanntmachung des Hinweises auf die Patenterteilung: **04.01.2017**
(21) Anmeldenummer: 08075879.0
(22) Anmeldetag: 13.11.2008
(51) Int. Cl.: A61B 1/06, H01L 33/50

(54) **Endoilluminator**
Endoilluminator
Endoilluminateur

(30) Priorität: 14.11.2007 DE 102007055003
(43) Veröffentlichungstag der Anmeldung: 20.05.2009
(73) Patentinhaber: Carl Zeiss Meditec AG, 07745 Jena (DE)
(72) Erfinder: Bausewein, Markus, 73431 Aalen (DE); Eichler, Michael, 73434 Aalen (DE)
(74) Vertreter: Theobald, Andreas

(56) Entgegenhaltungen:
- EP-A2- 1 467 416
- US-A- 6 028 694
- US-A1- 2004 135 158
- US-A1- 2005 154 262
- US-A1- 2005 194 876
- US-A1- 2006 069 314
- US-A1- 2007 018 177

## Beschreibung

Die vorliegende Erfindung betrifft einen Endoilluminator mit einem stabförmigen oder röhrenförmigen Körper, der ein proximales und ein distales Ende aufweist und der in eine kleindimensionierte Öffnung einführbar ist.

Derartige Endoilluminatoren finden bei Operationen Verwendung, bei denen die Beleuchtung sehr nahe an den Operationssitus herangeführt werden soll und/oder in das Körperinnere eingeführt werden soll. Insbesondere im Rahmen von minimalinvasiven Operationen sind Endoilluminatoren von Bedeutung. So wird beispielsweise in der Ophthalmologie, also der Augenheilkunde, das Innere des Auges oft durch einen kleinen Einschnitt hindurch mit Hilfe des dünnen Lichtleiters eines Endoilluminators beleuchtet. Ein medizinischer Illuminator, mit dem das Augeninnere beleuchtet werden kann und der eine an einem Handstück angeordnete dünne Kanüle zum Einführen in das Auge umfasst, ist beispielsweise in EP 1 719 482 A1 beschrieben. In dem darin beschriebenen Illuminator wird Licht aus einer Lichtquelle mittels eines sich durch die Kanüle erstreckenden Lichtleiters zum distalen Ende der Kanüle geführt. Am proximalen Ende der Kanüle ist ein Handstück vorhanden, von dem aus der Lichtleiter zu einer ortsfesten Lichtquelle weitergeführt ist.

Aus US2004/0090796A1 ist eine Lichtquelle zur Verwendung in der Ophthalmologie beschrieben, bei der die Lichtquelle statt in einer externen Einheit im Handstück angeordnet ist. Als Lichtquelle kommt eine LED zur Anwendung, deren Licht über eine Linse in das proximale Ende einer nadelartigen Faseroptik eingekoppelt wird. Das eingekoppelte Licht wird durch die Nadel zum distalen Ende geleitet. Das distale Ende der Nadel kann durch eine kleine Öffnung in das Auge eingeführt werden, um das Auge von Innen zu beleuchten. Die US2004/0090796A1 beschreibt außerdem eine weitere Ausführungsvariante, in der die LED im Inneren der Nadel kurz vor deren distalem Ende angeordnet ist. Eine elektrische Zuleitung ist durch die Nadel bis zur LED geführt. Wie diese Ausgestaltung konkret realisiert werden soll, ist in diesem Dokument jedoch nicht beschrieben. Insbesondere ist dem Dokument nicht zu entnehmen, wie eine LED in dem sehr begrenzten Rauminneren der Nadel angeordnet werden kann.

Die US 2005/0154262 A1 beschreibt ein Bilderfassungssystem für ein Endoskop. Zu Beleuchtungszwecken kann am distalen Ende des Endoskops eine Anordnung vorhanden sein, die eine oder mehrere Leuchtdioden enthält. Mittels eines Konverterleuchtstoffes an einem am distalen Ende des Endoskops befindlichen Kappenelement kann blaues Licht der Leuchtdioden in weißes Licht umgewandelt werden. Die Leuchtdioden sind auf thermisch leitfähige Flecken aufgebracht und werden über benachbarte elektrische Zuführungen mit elektrischer Spannung versorgt. Die einzelnen LEDs sind über Bonddrähte mit diesen Zuführungen verbunden.

Die US 2006/0069314 A1 beschreibt eine Festkörperbeleuchtungsvorrichtung für die Endoskopie, in der an einem distalen Ende Leuchtdioden angeordnet sind. Die Leuchtdioden sind über Kabel an eine Stromversorgung angeschlossen, die wiederum über flexible Schaltkreise mit Strom versorgt werden kann. GDie LEDs können mit einem Konverterleuchtstoff beschichtet sein, so dass sie weißes Licht abgeben.

Weitere dem Stand der Technik entsprechende Beleuchtungsvorrichtungen sind in US 6,028,694 und EP 1 467 416 A2 offenbart.

Die US 2004/0135158 A1 und die US 2007/0018177 A1 beschreiben vertikale LED's. Solche vertikalen LED's benötigen nur einen Bonddraht, da sie mit einem Anschluss direkt auf eine Kontaktfläche aufgebracht werden können.

Aufgabe der vorliegenden Erfindung ist es daher, einen Endoilluminator zur Verfügung zu stellen, der einen stabförmigen oder röhrenförmigen Körper mit einem proximalen

Ende und einem distalen Ende aufweist, wobei die Lichtquelle im Bereich des distalen Endes angeordnet ist, und auch mit kleinen Abmessungen des stabförmigen oder röhrenförmigen Körpers realisierbar ist.

Diese Aufgabe wird durch einen Endoilluminator nach Anspruch 1 gelöst. Die abhängigen Ansprüche enthalten vorteilhafte Ausgestaltungen der Erfindung. Die Merkmale der abhängigen Ansprüche können sowohl einzeln als auch in Kombination mit den Merkmalen anderer abhängiger Ansprüche vorteilhaft sein.

Ein Endoilluminator umfasst einen LED-Chip und einen stabförmigen Körper, welcher ein proximales Ende und ein distales Ende umfasst. Außerdem umfasst der stabförmige Körper wenigstens einen distalen hohlen Abschnitt, der vor dem distalen Ende beginnt und bis zum distalen Ende reicht und einen Zuleitungsabschnitt, der am proximalen Ende beginnt und bis zum distalen hohlen Abschnitt reicht. Hierbei kann der distale hohle Abschnitt insbesondere auch am proximalen Ende des stabförmigen Körpers beginnen, sodass der distale hohle Abschnitt und der Zuleitungsabschnitt von ein und dem selben hohlen Abschnitt gebildet sind. Der LED-Chip ist im distalen hohlen Abschnitt des stabförmigen Körpers angeordnet und wird über den Zuleitungsabschnitt mit Energie versorgt. Hierbei kann eine Leitung durch den Zuleitungsabschnitt des stabförmigen Körpers gelegt sein. Alternativ kann der stabförmige Körper selbst auch als elektrische Leitung ausgebildet sein. In dem Endoilluminator ist zwischen dem LED-Chip und dem distalen Ende des stabförmigen Körpers oder am distalen Ende selbst ein Konverterleuchtstoff angeordnet. Die Konvertereigenschaften des Konverterleuchtstoffes sind hinsichtlich des von dem LED-Chip emittierten Lichtes derart ausgewählt, dass er das von dem LED-Chip emittierte Licht in Licht einer gewünschten Wellenlängenverteilung, beispielsweise in weißes Licht, konvertiert.

In dem Endoilluminator weist der Zuleitungsabschnitt an seinem dem distalen hohlen Abschnitt zugewandten Ende eine Kontaktanordnung mit einem ersten Kontakt und einem zweiten Kontakt für den LED-Chip auf. Der LED-Chip ist dann so auf dem ersten Kontakt angeordnet, dass er diesen kontaktiert, und mit dem zweiten Kontakt durch einen Bonddraht verbunden. Diese Art der Kontaktierung benötigt nur wenig Raum, da lediglich ein Bonddraht vorhanden sein muss. Der elektrische Kontakt des LED-Chips mit dem ersten Kontakt kann mittels des so genannten Die-Bonden erfolgen, bei dem ein Chip elektrisch leitend ohne Gehäuse direkt auf ein Substrat aufgebracht wird.

In Bezug auf den Zuleitungsabschnitt ist der erste Kontakt ein Mittelkontakt und der zweite Kontakt ein Randkontakt sein. Diese Ausgestaltung hat den Vorteil, dass der LED-Chip, der ja auf dem ersten Kontakt diesen kontaktierend aufgebracht wird, auf der Mittelachse des stabförmigen Körpers angeordnet ist, was sich insbesondere als vorteilhaft erweist, wenn die Abstrahlung symmetrisch zur Mittelachse des stabförmigen Körpers erfolgen soll. Wenn zudem der Randkontakt den Mittelkontakt ringförmig umgibt, kann die Orientierung des Bonddrahts in Bezug auf die Radialrichtung des stabförmigen Körpers frei gewählt werden, was die Freiheit beim Orientieren des LED-Chips erhöht.

In medizinischen Beleuchtungseinrichtungen soll üblicherweise weißes Licht als Beleuchtungslicht zur Anwendung kommen. Weiße LEDs beruhen jedoch nicht auf einem Chip, der weißes Licht abstrahlt, sondern auf einem Chip, der blaues oder ultraviolettes Licht abstrahlt und dessen Licht durch ein auf dem LED-Chip aufgebrachtes Konvertermaterial in weißes Licht konvertiert wird. Durch das Aufbringen des Konvertermaterials auf dem LED-Chip vergrößern sich dessen Abmessungen im Vergleich zu einem LED-Chip ohne Konvertermaterial. Durch die Trennung von LED-Chip und Konvertermaterial der spärlich vorhandene Raum im distalen hohlen Abschnitt eines dünnen stabförmigen Körpers besser ausgenutzt werden, als wenn der LED-Chip und das Konvertermaterial eine Einheit bilden.

In dem Endoilluminator besteht die Möglichkeit, zuerst den LED-Chip mit der elektrischen Zuleitung zu kontaktieren, und dann erst das Konvertermaterial zwischen dem LED-Chip und dem distalen Ende des stabförmigen Körpers oder am distalen Ende selbst anzuordnen. Auf diese Weise wird das Kontaktieren und das Positionieren des LED-Chips nicht durch ein bereits zuvor auf den Chip aufgebrachtes Konvertermaterial beeinträchtigt. Die dadurch gewonnene erhöhte Flexibilität beim Kontaktieren und Positionieren des LED-Chips erleichtert seinen Einbau in den distalen hohlen Abschnitt eines dünnen stabförmigen Körpers. Außerdem weist der LED-Chip während des Einbaus auch geringere Abmessungen auf als eine fertige LED, die bereits von einem Konvertermaterial umgeben ist. Auch dies erhöht die Flexibilität beim Positionieren des LED-Chips und beim Anordnen der Kontakte für das Kontaktieren des LED-Chips.

In einer ersten konkreten Ausführungsvariante des Endoilluminators umgibt der Konverterleuchtstoff den LED-Chip in Form eines Vergusses. Dieser wird nach dem Positionieren und Kontaktieren des LED-Chips aufgebracht und kann insbesondere auch die Kontakte bedecken. Dies eröffnet die Möglichkeit, die Kontakte näher an den LED-Chip heranzuführen, als dies bei einer fertigen LED möglich wäre, also wenn der LED-Chip von vorneherein mit einem Konvertermaterial umgeben wäre. Außerdem kann beim Vergießen der vorhandene Raum optimal mit Konvertermaterial gefüllt werden, was bei einer vorgegebenen Geometrie einer fertigen LED nicht ohne Weiteres möglich wäre.

In einer alternativen Variante für eine konkrete Ausgestaltung des Endoilluminators ist der stabförmige Körper an seinem distalen Ende mit einer lichtdurchlässigen Scheibe verschlossen, auf die der Konverterleuchtstoff aufgebracht oder in die der Konverterleuchtstoff eingebracht ist. Das Aufbringen des Konverterleuchtstoffs auf die Scheibe kann beispielsweise in Form einer Folie oder einer Beschichtung realisiert werden, wobei die Folie beziehungsweise die Beschichtung grundsätzlich auf der Innenseite oder auf der Außenseite der Scheibe aufgebracht sein kann. Insbesondere ist es auch möglich, die Folie oder die Beschichtung sowohl auf der Außenseite als auch auf der Innenseite aufzubringen. Besonders vorteilhaft ist es jedoch, wenn die Folie beziehungsweise die Beschichtung nur auf der Innenseite der Scheibe, also der dem Inneren des distalen hohlen Abschnitts zugewandten Seite der Scheibe, angeordnet ist, da dann nicht auf die Bioverträglichkeit des Konvertermaterials geachtet werden muss und die Folie vor Körperflüssigkeiten geschützt ist, wenn der distale hohle Abschnitt hinreichend abgedichtet ist.

Bei Verwendung einer Folie zum Konvertieren kann das Folienmaterial selber der Konverterleuchtstoff sein, oder der Konverterleuchtstoff kann in ein Folienmaterial als Trägermaterial eingebracht sein.

Wenn der Konverterleuchtstoff in die Scheibe eingebracht ist, kann dies durch eine Dotierung realisiert sein. Gleiches gilt natürlich, wenn das Konvertermaterial in eine Trägerfolie eingebracht ist..

In einer besonderen Ausgestaltung der Ausführungsvariante mit Mittelkontakt und Randkontakt, insbesondere mit Mittelkontakt und ringförmigem Randkontakt, steht der Randkontakt in Richtung auf das distale Ende des stabförmigen oder röhrenförmigen Körpers über den Mittelkontakt vor. Zudem umgibt der Ringkontakt den Mittelkontakt spaltfrei, wobei zwischen dem Ringkontakt und dem Mittelkontakt eine elektrische Isolierung vorhanden ist. In dieser Konfiguration bildet der Mittelkontakt eine Vertiefung, die vom Randkontakt umgeben ist und als eine Aufnahme für Konvertermaterial dienen kann. Das Konvertermaterial kann hierbei als Verguss in die Vertiefung eingebracht werden. Es ist aber auch möglich, ein pulverförmiges Konvertermaterial in die Vertiefung einzubringen und anschließend durch eine geeignete Behandlung, beispielsweise mittels eines geeigneten Aushärteverfahrens, in der Vertiefung zu fixieren. Aber auch das mechanische Fixieren etwa eines flüssigen oder pulverförmigen Konvertermaterials mittels einer die Vertiefung dicht abschließenden Scheibe ist grundsätzlich möglich. Die elektrische Isolierung zwischen dem Mittelkontakt und dem Randkontakt kann insbesondere durch eine Klebeverbindung mit einem elektrisch isolierenden Kleber realisiert sein.

Der distale hohle Abschnitt des Endoilluminators braucht nicht einstückig mit den übrigen Abschnitten den stabförmigen Körpers ausgebildet zu sein. Es besteht auch die Möglichkeit, dass der distale hohle Abschnitt von einer zylinderförmigen Hülse gebildet ist, in die der LED-Chip eingebracht ist, und die auf den Zuleitungsabschnitt des stabförmigen Körpers aufgesetzt ist. Der LED-Chip kann dann zuerst in die Hülse eingesetzt werden, bevor diese auf das distale Ende des Zuleitungsabschnitts aufgesetzt wird. Insbesondere ist es hierbei möglich, den LED-Chip erst mit Kontakten des Zuleitungsabschnittes zu verbinden und gegebenenfalls die LED zu vergießen, bevor die Hülse über die LED gestülpt wird. Dabei kann der Konverterverguss in dieser Ausführungsvariante bereits ausgehärtet sein, wenn die Hülse aufgesetzt wird, sodass grundsätzlich nichts dagegen spricht, die Hülse so aufzusetzen, dass sie den Konverterverguss mit einer am distalen Ende angeordneten Frontscheibe kontaktiert, wodurch sich eine Reproduzierbarkeit der Positionierung des LED-Chips im Herstellungsprozess für den Endoilluminator einfach erreichen lässt. Die zuvor beschriebene Kontaktanordnung kann hierbei einen Sockel bilden, auf den die Hülse aufgesetzt ist.

Vorteilhafterweise weist der Stab eine gute Wärmeleitfähigkeit auf, beispielsweise dadurch, dass er als Metallstab ausgebildet ist. Als Metallstab soll in diesem Zusammenhang auch ein Draht angesehen werden, da die Unterscheidung zwischen einem Metallstab und einem Metalldraht bei sehr dünnen Metallstäben verschwimmt.

Wenigstens der LED-Chip kann in allen Ausführungsvarianten insbesondere auch mit einem sterilisierbaren Material, beispielsweise Epoxid, Acrylat oder Silikon gekapselt sein. Diese Kapselung kann beispielsweise durch den stabförmigen Körper realisiert sein, wenn dessen Wände aus entsprechendem Material hergestellt sind.

Weitere Merkmale, Eigenschaften und Vorteile ergeben sich aus der nachfolgenden Beschreibung von Ausführungsbeispielen unter Bezugnahme auf die beiliegenden Figuren.
Figur 1 zeigt in stark schematisierter Darstellung einen Endoilluminator.
Figur 2 zeigt eine Abwandlung des in Figur 1 dargestellten Endoilluminators.
Figur 3 zeigt das distale Ende des Endoilluminators aus Figur 1 oder Figur 2.
Figur 4 zeigt eine alternative Ausgestaltung des distalen Endes des Endoilluminators.
Figur 5 zeigt eine weitere alternative Ausgestaltung des distalen Endes des Endoilluminators.
Figur 6 zeigt eine Ausgestaltung des distalen Endes des Endoilluminators.
Figur 7 zeigt eine alternative Ausgestaltung des distalen Endes des Endoilluminators.

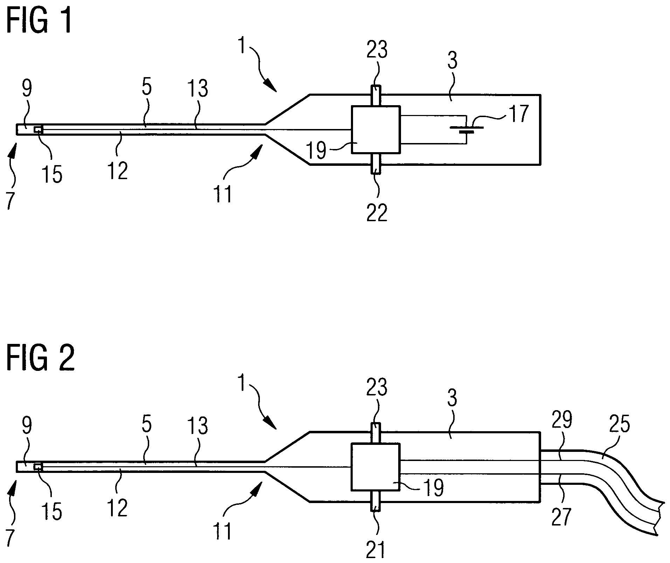

Nachfolgend wird ein Endoilluminator beschrieben. Der Endoilluminator 1 ist in Figur 1 in einer stark schematisierten Darstellung gezeigt. Er umfasst einen Handgriff 3 sowie ein vom Handgriff 3 ausgehendes Röhrchen beziehungsweise Stäbchen 5, welches vorzugsweise einen Außendurchmesser von nicht mehr als 1 mm und vorzugsweise von nicht mehr als 0,5 mm aufweist. Das distale Ende des Röhrchens beziehungsweise Stäbchens 5 kann auch eine Spitze aufweisen, so dass das Röhrchen eine Nadel bildet.

Am distalen Ende 7 des Röhrchens beziehungsweise Stäbchens 5 ist ein distaler hohler Abschnitt 9 vorhanden, der vor dem distalen Ende 7 beginnt und bis zu diesem reicht. In Richtung auf das proximale Ende 11 des Röhrchens beziehungsweise Stäbchens 5 schließt sich ein Zuleitungsabschnitt 13 an den distalen hohlen Abschnitt 9 an. Durch diesen Zuleitungsabschnitt 13 ist in der in Figur 1 dargestellten Ausführungsvariante des Endoilluminators 1 eine elektrische Zuleitung 15 bis zum distalen hohlen Abschnitt 9 geführt. Im distalen hohlen Abschnitt ist ein Leuchtdiodenchip (LED-Chip) angeordnet, der von der elektrischen Leitung 15 mit elektrischer Leistung versorgt wird.

Im Bereich des distalen hohlen Abschnitts 9 ist die Wand des Röhrchens beziehungsweise Stäbchens 5 zumindest teilweise transparent. Zwischen dem LED-Chip 15 und zumindest dem transparenten Abschnitt der Wand des distalen hohlen Abschnitts 9 ist ein in Figur 1 nicht dargestelltes Konvertermaterial angeordnet, welches das vom LED-Chip emittierte Licht, im Folgenden Anregungsstrahlung genannt, wenigstens teilweise in Licht einer längeren Wellenlänge konvertiert.

Typischerweise findet als LED-Chip 15 ein LED-Chip Verwendung, der blaues, violettes oder ultraviolettes Licht emittiert. Dies ist vorteilhaft, weil von Konverterleuchtstoffen konvertiertes Licht immer eine längere Wellenlänge als das Anregungslicht hat. Je kurzwelliger die Anregungsstrahlung ist, desto größer ist der Wellenlängenbereich im sichtbaren Spektralbereich, in den konvertiert werden kann.

Wenn beispielsweise der LED-Chip 15 blaues Licht emittiert und der Konverterleuchtstoff so gewählt ist, dass ein Teil dieses blauen Lichtes in gelbes Licht konvertiert wird, so erscheint das Licht, das aus der Überlagerung der konvertierten Komponente der Anregungsstrahlung und der nicht konvertierten Komponente der Anregungsstrahlung entsteht, weiß. Auf diese Art kann trotz der Verwendung eines blaues, violettes oder ultraviolettes Licht emittierenden LED-Chips vom Endoilluminator 1 weißes Licht abgegeben werden. Wenn mehr als ein Konvertermaterial vorhanden ist, besteht zudem die Möglichkeit, Teile der Anregungsstrahlung in Licht verschiedener Wellenlängen zu konvertieren, beispielsweise in grünes, gelbes und rotes Licht. Zusammen mit einem nicht konvertierten Anteil der Anregungsstrahglung lässt sich so eine breite und gleichmäßige Wellenlängenverteilung des vom distalen Ende 7 des Endoilluminators 1 emittierenden Lichtes erreichen.

Um Schädigungen des Gewebes durch nicht konvertierte Anregungsstrahlung zu vermeiden, kann die Wand des distalen hohlen Abschnittes dort, wo sie transparent ist, zudem mit einer für UV-Strahlung undurchlässigen Beschichtung versehen sein. Alternativ zu einer Beschichtung kann ein UV-undurchlässiges Material auch in das transparente Material des distalen hohlen Abschnittes 9 eingebracht sein, oder das transparente Material ist selbst UV-undurchlässig.

Der Zuleitungsabschnitt 12 ist im vorliegenden Beispiel als Röhrchen ausgebildet, durch den die elektrische Leitung 13 zum distalen hohlen Abschnitt 9 geführt ist. Im Handgriff 3 befindet sich im vorliegenden Ausführungsbeispiel eine Batterie, die in Figur 1 als Spannungsquelle 17 dargestellt ist und über die der LED-Chip 15 mit elektrischer Leistung versorgt wird. Zwischen der Batterie 17 und der elektrischen Leitung 13 ist eine Elektronik 19 angeordnet, auf die beispielsweise über Tasten 21, 23 eingewirkt werden kann, etwa zum Ein- und Ausschalten des Endoilluminators 1 oder zum Regeln seiner Helligkeit. Statt der Tasten können selbstverständlich auch andere Betätigungselemente, beispielsweise Schieber, Rädchen, etc. vorhanden sein.

Obwohl der Zuleitungsabschnitt 12 im vorliegenden Ausführungsbeispiel als Röhrchen ausgebildet ist, kann er auch als Stäbchen ausgebildet sein. In diesem Fall ist das Stäbchen aus zwei Bestandteilen zusammengesetzt, die gegeneinander elektrisch isoliert sind. Diese beiden Bestandteile dienen dann als elektrische Zuleitungen für den LED-Chip 15. Ein derartiges Stäbchen kann zudem mit einer isolierenden Schicht umgeben sein. Vorzugsweise ist diese Schicht gut bioverträglich und sterilisierbar. Falls die Schicht für sichtbares Licht transparent ist, kann sie zudem auch den distalen hohlen Abschnitt 9 umgeben, sodass die Schicht eine geschlossene Schicht für das gesamte Stäbchen beziehungsweise Röhrchen 5, also einschließlich des Zuleitungsabschnitts 12 und des distalen hohlen Abschnitts 9, bildet.

Eine alternative Variante des in Figur 1 dargestellten Endoilluminators zeigt Figur 2. Der in Figur 2 dargestellte Endoilluminator unterscheidet sich von dem in Figur 1 dargestellten Endoilluminator lediglich dadurch, dass er statt einem Batteriefach ein Kabel 25 aufweist, durch welches von der Elektronik 19 ausgehende elektronische Leitungen 27, 29 zu einer externen Stromversorgung geführt werden. Die übrigen Elemente des in Figur 2 dargestellten Endoilluminators entsprechen denen des in Figur 1 dargestellten Endoilluminators. Sie sind daher mit den selben Bezugsziffern wie in Figur 1 bezeichnet und werden nicht noch einmal erläutert.

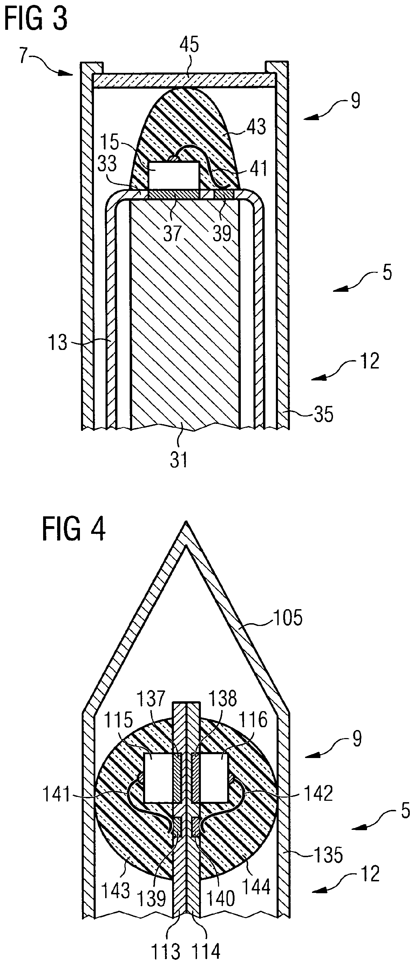

Figur 3 zeigt das distale Ende einer ersten Variante des Stäbchens beziehungsweise Röhrchens 5, das in der Ausführungsvariante als Röhrchen 35 ausgebildet ist im Detail. In der Figur sind das distale Ende 7, der distale hohle Abschnitt 9 mit darin angeordnetem LED-Chip 15 sowie der Zuleitungsabschnitt 12 mit der darin verlaufenden elektrischen Leitung 13 zu erkennen.

Die elektrische Leitung 13 ist in dieser Ausführungsvariante als flexibles Leiterband ausgestaltet, welches um ein Stäbchen 31 mit abgeflachtem distalen Ende 33 herumgeführt ist. Das Stäbchen 31 sowie das Leiterband 13 sind in ihren Abmessungen so gewählt, dass sie gemeinsam in das Röhrchen 35 eingeführt werden können, wenn das Leiterband 13 um das Stäbchen 31 herumgeführt ist.

In dem Bereich, in dem das Leiterband 13 am flachen distalen Abschnitt 33 des Stäbchens 31 anliegt, sind ein erster elektrischer Kontakt 37 und ein zweiter elektronischer Kontakt 39 im Leiterband 13 angeordnet. Auf den ersten elektrischen Kontakt 37 ist der LED-Chip 15 den Kontakt 37 elektrisch kontaktierend aufgebracht. Mit dem zweiten, vom ersten elektrischen Kontakt isolierten, elektrischen Kontakt 39 ist der LED-Chip 15 über einen Bonddraht 41 verbunden.

Der LED-Chip 15 und der Bonddraht 41 sind außerdem über einen auf dem Leiterband 13 aufgebrachten Verguss 43 gekapselt. Dem Verguss 43 ist im vorliegenden Ausführungsbeispiel zudem ein Konvertermaterial beigemischt, welches die vom LED-Chip 15 ausgehende Anregungsstrahlung wenigstens teilweise in Licht mit einer längeren Wellenlänge konvertiert, wie dies mit Bezug auf Figur 1 beschrieben worden ist. Selbstverständlich können dem Verguss 43 auch mehrere verschiedene Konvertermaterialien beigemischt sein. Es besteht grundsätzlich aber auch die Möglichkeit, dass das Vergussmaterial selbst ein Konvertermaterial ist.

Der distale hohle Abschnitt 9 ist am distalen Ende 7 des Röhrchens 135 von einer Scheibe 45 abgeschlossen. Die Scheibe 45 ist wenigstens im sichtbaren Spektralbereich durchlässig, kann jedoch auch in angrenzenden Spektralbereichen durchlässig sein. Vorzugsweise ist sie jedoch im ultravioletten Spektralbereich undurchlässig, um eventuelle ultraviolette Strahlungskomponenten des LED-Chips 15, die vom Konvertermaterial nicht konvertiert worden sind, abzublocken.

Statt im Verguss 43 kann das Konvertermaterial auch in oder auf der transparenten Scheibe 45 aufgebracht sein. Falls es auf der transparenten Scheibe 45 aufgebracht ist, kann es entweder in Form einer Folie oder in Form einer Beschichtung aufgebracht sein. Die Folie kann hierbei entweder selbst das Konvertermaterial bilden oder als Trägermaterial für ein in die Folie eingebrachtes Konvertermaterial dienen. Wenn das Konvertermaterial in die Folie oder die Scheibe 45 eingebracht ist, kann dies in Form einer Dotierung realisiert sein. Grundsätzlich besteht auch die Möglichkeit, sowohl einen Konverterleuchtstoff im Verguss 43 als auch in oder auf der Scheibe 45 anzuordnen. Beispielsweise kann hierbei das Konvertermaterial der Scheibe 45 das Anregungslicht in eine andere Wellenlänge konvertieren als das Konvertermaterial des Vergusses 43.

Im vorliegenden Beispiel dichtet die Scheibe 45 außerdem den distalen hohlen Abschnitt 9 gegen die Umgebung ab. Diese Dichtfunktion ist jedoch nicht nötig, wenn der distale hohle Abschnitt einen dichtenden Überzug, wie er mit Bezug auf Figur 1 beschrieben worden ist, aufweist.

Zum Montieren der in Figur 3 dargestellten Variante des Endoilluminators und insbesondere des Stäbchens beziehungsweise Röhrchens 5 wird zuerst der LED-Chip 15 so auf den ersten Kontakt 37 des Leiterbandes 13 aufgesetzt, dass eine elektrisch leitende Verbindung entsteht, und die Bondverbindung mit dem zweiten Kontakt 39 hergestellt. Danach wir die Anordnung vergossen. Das Vergießen kann auf einem ebenen Untergrund stattfinden, sodass die Rückseite des Leiterbandes 13 nach dem Vergießen und Aushärten des Vergusses eine ebene Fläche darstellt. Anschließend wird das Leiterband mit dem vergossenen Bereich an das flache distale Ende 33 des Stäbchens 31 angelegt und an diesem beispielsweise durch Verkleben fixiert. Die Enden des Leiterbandes 13 werden dann entlang dieses Röhrchens 31 geführt und gegebenenfalls mit diesem ebenfalls verklebt. Die gesamte Anordnung wird dann in das hohle Röhrchen 35 eingeführt. Das Einführen kann soweit erfolgen, dass der Verguss 43 die Scheibe 45 kontaktiert.

Diese Vriante bietet den Vorteil, dass die Fertigung bis einschließlich des Aufbringens des Vergusses 43 mit einem ebenen Leiterband erfolgen kann. Um das Einführen der Anordnung aus Stäbchen 31 und Leiterband 13 mit dem LED-Chip 15 in das Röhrchen 35 zu erleichtern, kann dieses am proximalen Ende eine Aufweitung aufweisen. Zudem kann das Leiterband 13 in denjenigen Bereichen, mit denen es aus dem proximalen Ende des Röhrchens 35 herausragt, verbreitert sein, sodass die Kontaktierung mit der Elektronik 19 erleichtert wird. Insbesondere können diese Enden dann direkt als Kontakte dienen.

Eine zweite Variante des Stäbchens beziehungsweise Röhrchens 5 des Endoilluminators 1 ist in Figur 4 dargestellt. In dieser Variante sind zwei flexible ebene Leiterbänder 113, 114 Rückseite an Rückseite miteinander verklebt. An ihren Vorderseiten sind die Leiterbänder 113, 114 mit ersten elektrischen Kontakten 137, 138 und zweiten elektrischen Kontakten 139, 140 ausgestattet. Auf jedem ersten elektrischen Kontakt 137, 138 ist ein LED-Chip 115, 116 den Kontakt elektrisch leitend kontaktierend angeordnet. Mit den zweiten Kontakten 139, 140 sind die LED-Chips 115, 116 jeweils über Bonddrähte 141, 142 verbunden. Die LED-Chips 115, 116 und die jeweiligen Bonddrähte 141, 142 sind zudem jeweils von einem Verguss 143, 144 umgeben, der auch wenigstens einen Konverterleuchtstoff enthalten kann.

Das Stäbchen beziehungsweise Röhrchen 5 ist in der vorliegenden Variante als transparentes Kunststoffröhrchen 135 mit Spitze 105 ausgeführt, durch dessen Zuleitungsabschnitt 12 die miteinander verklebten Leiterbänder 113, 114 bis in den distalen hohlen Abschnitt 9 geführt sind. Die LED-Chips 115, 116 sind dabei so an den Leiterbändern 113, 114 angeordnet, dass sie sich nach dem Einschieben im distalen hohlen Bereich 9 befinden.

Anstatt im Verguss 143, 144 kann das Konvertermaterial auch auf das Röhrchen 135 aufgebracht sein oder in das transparente Wandmaterial des Röhrchens 135 eingebracht sein. Hinsichtlich der für das Einbeziehungsweise Aufbringen des Konverterleuchtstoffes vorhandenen Optionen gilt das mit Bezug auf Figur 3 ausgeführte analog, wobei jedoch die transparente Scheibe 45 in der vorliegenden Variante durch das transparente Material des Röhrchens 135 ersetzt ist.

Es sei noch angemerkt, dass nicht notwendigerweise das gesamte Röhrchen 135 transparent ausgestattet sein muss. Es reicht, wenn es im distalen hohlen Abschnitt 9 transparent ausgestaltet ist. Das transparente Material des Röhrchens 135 kann zudem so ausgestaltet sein, dass es für etwaige ultraviolette Komponenten des Anregungslichtes der LEDs 115, 116 undurchlässig ist.

Anstatt des mit Bezug auf Figur 4 beschriebenen Röhrchens 135 kann in dieser Variante jedoch auch grundsätzlich ein Röhrchen 35 Verwendung finden, wie es mit Bezug auf Figur 3 beschrieben worden ist, wobei aufgrund der Anordnung der LED-Chips 115, 116 jedoch eine transparente Umfangsfläche im Bereich des distalen hohlen Abschnittes 9 vorteilhaft ist.

Durch das Vorhandensein von zwei LED-Chips ermöglicht diese Ausgestaltung eine große Helligkeit des vom Endoilluminator 1 abgegebenen Lichtes. Zudem sind außer den Leiterbändern mit den darauf angeordneten LED-Chips keine weiteren Komponenten durch den Zuleitungsabschnitt 12 des Röhrchens 135 zu führen. Das Aufbringen der LED-Chips 115, 116 und das Vergießen kann im vorliegenden Ausführungsvariante auf dieselbe Weise erfolgen, wie es im Rahmen der ersten Variante beschrieben worden ist.

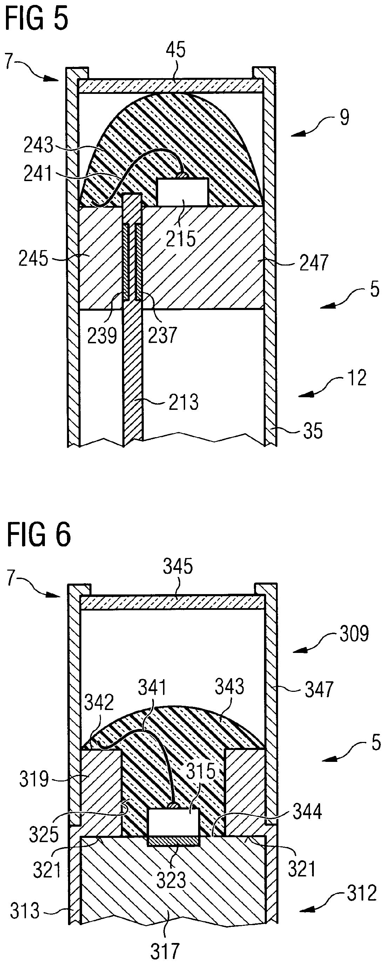

Eine dritte Variante für das Stäbchen beziehungsweise Röhrchen 5 des Endoilluminators 1 ist in Figur 5 dargestellt. In dieser Variante findet ein Röhrchen 35 derselben Art Verwendung, wie es mit Bezug auf die erste Ausführungsvariante beschrieben worden ist. In dieses Röhrchen 35 ist eine Kontaktanordnung 245, 247 eingebracht, auf der ein LED-Chip 215 angeordnet ist. Die Kontaktanordnung 245, 247 wird über ein flexibles Leiterband 213, das durch den hohlausgeführten Zuleitungsabschnitt 12 geführt ist, mit Strom versorgt. Hierzu weist das flexible Leiterband an seinem distalen Ende zwei elektrische Kontaktierungsabschnitte 237, 239 auf, die an voneinander abgewandten Seiten des Leiterbandes 213 angeordnet sind.

Die Kontaktanordnung 245, 247 umfasst einen ersten elektrisch leitenden Block 247 und einen zweiten elektrisch leitenden Block 245, die miteinander verbunden und gegeneinander elektrisch isoliert sind. Das Leiterband 213 erstreckt sich durch die Kontaktanordnung 245, 247 hindurch und zwar so, dass der erste elektrisch leitende Block 247 den ersten elektrischen Kontaktbereich 237 des Leiterbandes 213 elektrisch kontaktiert, während der zweite elektrisch leitende Block 245 den zweiten elektrischen Kontaktierungsabschnitt 239 des Leiterbandes 213 kontaktiert. Der LED-Chip 215 ist direkt auf dem ersten elektrisch leitenden Block 247 diesen elektrisch kontaktierend angeordnet. Die Verbindung des LED-Chips 215 mit dem zweiten elektrisch leitenden Block 245 der Kontaktanordnung ist durch einen Bonddraht 241 hergestellt.

Die gesamte Anordnung aus LED-Chip 215 und Bonddraht ist von einem Verguss 243 umgeben. Wie in den übrigen Varianten kann der Verguss 243 und/oder die Scheibe 45 mit wenigstens einem Konvertermaterial versehen sein. Zudem kann die Scheibe 45 so ausgestaltet sein, dass sie für ultraviolette Komponenten des Anregungslichtes undurchlässig ist.

Das Herstellen der Kontaktanordnung mit dem darauf angeordneten LED-Chip und dem Verguss kann wie folgt erfolgen: Zuerst werden die Blöcke 245, 247 um das Leiterband 213 herum angeordnet und miteinander verklebt. Das Verkleben erfolgt dabei so, dass die Blöcke 245, 247 gegeneinander isoliert sind. Dann wird der LED-Chip 215 auf den ersten Block 247 aufgesetzt und anschließend mittels eines Bonddrahtes 241 mit dem zweiten Block 245 verbunden. Danach wird ein Verguss auf diejenige Seite der Kontaktanordnung 245, 247 aufgebracht, auf der sich der LED-Chip 215 und der Bonddraht 241 befinden. Anschließend kann die Kontaktanordnung mit dem darauf angeordneten und vergossenen LED-Chip 215 auf Maß gedreht werden, sodass die Anordnung genau in das Röhrchen 35 hineinpasst. Schließlich wird die Anordnung in das Röhrchen eingeführt.

Eine erste Ausführungsvariante des Stäbchens beziehungsweise Röhrchens 5 ist in Figur 6 dargestellt. Es sind wie in den Figuren 3 bis 5 der distale hohle Abschnitt 309 sowie ausschnittsweise der Zuleitungsabschnitt 312 dargestellt. Im Unterschied zu den mit Bezug auf die Figuren 3 bis 5 beschriebenen Varianten besitzt Zuleitungsabschnitt 312 der mit Bezug auf Figur 6 dargestellten Ausführungsvariante nicht in Form eines Röhrchens, sondern in Form eines Stäbchens. Das Stäbchen ist aus zwei Komponenten aufgebaut, nämlich einer zylinderförmigen Hülse und einem in die zylinderförmige Hülse 313 eingeführten und mit dieser verklebten Stift 317.

Der Stift 317 und die Hülse 313 sind aus einem elektrisch leitenden Material, beispielsweise Metall, hergestellt und gegeneinander elektrisch isoliert.

Entweder die Innenseite der Hülse 313 oder die Außenseite des Stiftes 317 oder beide weisen eine elektrische Isolierung auf, sodass beide in körperlichem Kontakt miteinander stehen können, ohne dass ein elektrischer Kontakt entsteht. Die Hülse 313 weist zudem an ihrem distalen Ende einen Abschnitt 319 auf, in der sowohl ihr Außendurchmesser als auch ihr Innendurchmesser verringert ist. Am Übergang zwischen dem Abschnitt 319 mit verringertem Außen- und Innendurchmesser und dem Abschnitt ohne verringertem Außen- und Innendurchmesser ist eine ringförmige Anschlagfläche 321 gebildet, die als Anschlag für den Stift 317 dient.

Am distalen Ende 344 des Stiftes 317 ist ein Kontaktflecken 323 vorhanden, in dem auf jeden Fall keine Isolierung vorhanden ist. Auf den Kontaktflecken 323 ist ein LED-Chip 315 aufgesetzt und mit dem Kontaktflecken 323 elektrisch leitend verbunden. Der LED-Chip 315 ist außerdem über einen Bonddraht 341 mit der Stirnfläche 342 desjenigen Abschnitts 319 der Hülse 313 verbunden, in dem die Hülse einen verringerten Innen- und Außendurchmesser aufweist. Zumindest diese Stirnfläche 342 weist keine elektrische Isolierung auf. Der Abschnitt 319 der Hülse 313 mit verringerten Innen- und Außendurchmesser bildet einen ringförmigen Kontakt, der um einen zentralen Kontakt herum, nämlich um den Kontaktflecken 323, angeordnet ist. Da er über das distale Ende 344 des Stiftes 317 vorsteht, begrenzt seine Innenwand 325 eine Vertiefung, die bis zum distalen Ende 344 des Stiftes 317 reicht. In diese Vertiefung ist ein Verguss 343 eingebracht. Diese Ausgestaltung bietet den Vorteil, dass der Verguss lediglich in die Vertiefung eingefüllt zu werden braucht.

Der Verguss kann in der Ausführungsvariante nach dem Aufbringen und Kontaktieren des LED-Chips in die Vertiefung eingefüllt und ausgehärtet werden. Statt durch Aushärten kann der Verguss auch durch eine transparente Abschlussscheibe in der Vertiefung gehalten werden, wenn der Bonddraht nicht die Stirnfläche 342 des Abschnitts 319, sondern eine Innenfläche dieses Abschnitts kontaktiert.

In der Ausführungsvariante ist der distale hohle Abschnitt 309 durch eine distale Hülse 347 gebildet, deren Innendurchmesser dem Außendurchmesser der Hülse 313 des Zuleitungsabschnittes im Abschnitt mit reduziertem Innen- und Außendurchmesser entspricht. Dadurch kann die distale Hülse 347 auf den Zuleitungsabschnitt aufgesetzt und beispielsweise mit diesem verklebt werden.

Das distale Ende der distalen Hülse 347 ist mit einer transparenten Scheibe verschlossen. Die Ausgestaltung der Scheibe entspricht der Ausgestaltung der mit Bezug auf Figur 3 beschriebenen Scheibe. Hinsichtlich der Anordnung des Konvertermaterials gilt das mit Bezug auf Figur 3 ausgeführte ebenfalls analog. Das heißt, das Konvertermaterial kann im Verguss, in der Scheibe oder auf der Scheibe angeordnet sein. Auch Kombinationen hiervon sind möglich, wobei auch eingeschlossen sein soll, dass auch mehrere Konvertermaterialien, die sich voneinander unterscheiden, vorhanden sein können.

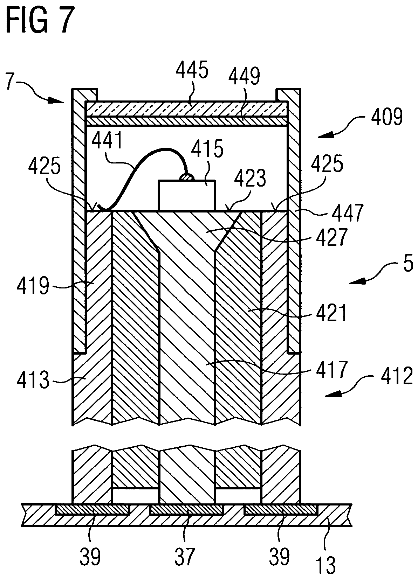

Eine zweite Variante des Stäbchens beziehungsweise Röhrchens 5 des Endoilluminators 1 ist in Figur 7 dargestellt. Diese Variante ähnelt der mit Figur 6 beschriebenen Variante insofern, als dass der Zuleitungsabschnitt 412 als Stäbchen ausgebildet ist. Er umfasst eine zylindrische Hülse 413 und ein im Zentrum der zylindrischen Hülse angeordnetes zentrales Stäbchen 417. Der Außendurchmesser des zentralen Stäbchens 417 ist geringer als der Innendurchmesser der Hülse 413. Zwischen beiden ist ein Isolatormaterial 421 vorhanden, welches das Stäbchen 417 mit Bezug auf die Hülse 413 in Position hält und eine elektrische Isolierung zwischen beiden sicherstellt. In der zweiten Ausführungsvariante erstreckt sich das Isolatormaterial 421 im Wesentlichen über die gesamte axiale Länge der Anordnung aus Hülse 413 und Stäbchen 417. Es ist aber auch möglich, das Isolatormaterial 421 lediglich in einigen axialen Abschnitten anzuordnen, solange die Stabilität der Anordnung und die elektrische Isolation gewahrt ist.

Im Unterschied zur in Figur 6 dargestellten ersten Ausführungsvariante liegen die distalen Stirnflächen 423, 425 des Stäbchens 417 beziehungsweise der Hülse 419 in einer gemeinsamen Ebene. Die distale Stirnfläche 423 des zentralen Stäbchens 417 bildet einen ersten Kontakt, auf den der LED-Chip 415 mit elektrisch leitender Kontaktierung aufgesetzt ist. Um die für das Aufsetzen des LED-Chips 415 vorhandene Fläche zu vergrößern, ist das zentrale Stäbchen 417 in seinem distalen Endbereich 427 etwas verbreitert, ohne dabei jedoch die elektrische Isolation zur Hülse 419 zu gefährden. Die distale Stirnfläche 425 der Hülse 419 bildet einen ringförmigen zweiten Kontakt, mit dem der LED-Chip 415 über einen Bonddraht 441 verbunden ist.

Das distale Ende des Zuleitungsabschnitts 412 weist einen Abschnitt 419 auf, in dem die Hülse 413 einen reduzierten Außendurchmesser aufweist. Eine distale Hülse 447 ist auf diesen Abschnitt 419 mit reduziertem Außendurchmesser aufgesetzt. Die Abmessungen der distalen Hülse 447 sind so gewählt, dass ihr Innendurchmesser dem Außendurchmesser des Abschnittes 419 entspricht und der Außendurchmesser der distalen Hülse 447 mit dem Außendurchmesser der Hülse 413 übereinstimmt, sodass eine möglichst glatte Außenfläche des Röhrchens beziehungsweise Stäbchens 5 entsteht.

Das distale Ende der distalen Hülse 447 ist mit einer Scheibe 445 verschlossen. Diese ist im sichtbaren Spektralbereich transparent und kann im ultravioletten Spektralbereich undurchlässig ausgestaltet sein. Zudem ist die Scheibe 445 an ihrer dem Hülseninneren zugewandten Seite mit einer Konverterbeschichtung 449 versehen. Diese Konverterbeschichtung kann entweder aus dem Konvertermaterial selbst bestehen oder das Konvertermaterial enthalten. Kombinationen aus wenigstens zwei Konvertermaterialien sind hierbei grundsätzlich auch möglich.

Obwohl in der vorliegenden zweiten Ausführungsvariante das Konvertermaterial in Form einer Beschichtung 449 auf die Innenseite der Scheibe 445 aufgebracht ist, kann es auch auf die Außenseite der Scheibe 445 aufgebracht oder beispielsweise als Dotierung in die Scheibe 445 eingebracht sein. Außerdem ist es auch in der in Figur 7 dargestellten Ausführungsvariante möglich, den LED-Chip 415 und den Bonddraht 441 zu vergießen. In diesem Fall kann auch der Verguss das Konvertermaterial beziehungsweise die Konvertermaterialien enthalten.

Es sei an dieser Stelle darauf hingewiesen, dass nicht nur die mit Bezug auf Figur 7 beschriebene Ausführungsvariante ohne Verguss herstellbar ist, sondern der Verguss auch bei den übrigen Varianten weggelassen werden kann. In diesem Fall ist das Konvertermaterial auf die Scheibe aufgebracht oder in die Scheibe eingebracht.

In den beiden Ausführungsvarianten, in denen eine distale Hülse Verwendung findet, die auf den Zuleitungsabschnitt aufgesetzt ist, ist es nicht nötig, ein Leiterband durch ein langes Röhrchen zu führen. Auch braucht kein flexibles Leiterband im Bereich des LED-Chips zum Einsatz zu kommen. Da zudem die zylinderförmige Hülse des Zuleitungsabschnittes eine Fassung für die distale Hülse bildet, ist eine exakte Positionierung der distalen Hülse in Bezug auf den Zuleitungsabschnitt möglich. Dies ist insbesondere dann vorteilhaft, wenn die Innenseite der Scheibe der distalen Hülse mit einer Konverterbeschichtung versehen ist, da mit einfachen Mitteln sichergestellt werden kann, dass die Scheibeninnenseite mit keinem Element im Inneren des distalen hohlen Abschnitts in Kontakt gerät. Bei einem Kontakt könnte nämlich die Konverterbeschichtung Schaden nehmen. Falls das Konvertermaterial in einen Verguss eingebracht worden ist, anstatt auf die Scheibe aufgebracht zu sein, ist ein Kontakt zwischen dem Verguss und der Scheibe jedoch unkritisch.

Figur 7 zeigt außerdem, wie die Hülse 413 und der zentrale Stift 417 mit Strom versorgt werden. Am proximalen Ende des Stiftes 417 und der Hülse 413 ist ein flexibles Leiterband 13 angeordnet, welches Kontaktierungsabschnitte 37 und 39 aufweist, die gegeneinander isoliert sind. Der zentrale Stift 417 kontaktiert dann den Kontaktierungsabschnitt 37, während die Hülse 413 den ringförmig um den Kontaktierungsabschnitt 37 herum angeordneten Kontaktierungsabschnitt 39 kontaktiert. Dieselbe Art der Kontaktierung ist auch in der mit Bezug auf Figur 6 dargestellten Ausführungsvariante möglich.

In den Ausführungsvarianten kann das Aufbringen des LED-Chips auf Kontaktflächen, Kontakte beziehungsweise Kontaktflecken durch sogenanntes Die-Bonden erfolgen. Das Die-Bonden ist eine sogenannte Chip-on-Board-Technologie, in der ein Chip ohne Gehäuse direkt auf ein Substrat, im vorliegenden Fall die Kontaktflächen, geklebt, gelötet, geglast, geschweißt (beispielsweise mittels Ultraschall) oder legiert wird, wobei eine elektrisch leitender Kotakt zwischen dem Substrat und dem Chip hergestellt wird.

Die Erfindung stellt Endoilluminatoren bereit, die auch bei Verwendung von Röhrchen beziehungsweise Stäbchen mit sehr geringem Außendurchmesser eine Anregungslichtquelle am distalen Ende aufweisen können. Dadurch entfällt das oft schwierige Einkoppeln des Lichtes in einen Lichtleiter.

## Patentansprüche

1. Endoilluminator (1), welcher umfasst:
- einen LED-Chip (315, 415) und
- einen stabförmigen oder röhrenförmigen Körper (5) mit einem proximalen Ende (11), einem distalen Ende (7), wenigstens einem distalen hohlen Abschnitt (9), der vor dem distalen Ende beginnt (7) und bis zum distalen Ende (7) reicht, und einem Zuleitungsabschnitt (12), der am proximalen Ende (11) beginnt und bis zum distalen hohlen Abschnitt (9) reicht,
wobei
- der LED-Chip (315, 415) im distalen hohlen Abschnitt des stabförmigen oder röhrenförmigen Körpers (5) angeordnet ist und über den Zuleitungsabschnitt (12) mit Energie versorgt wird,
- ein Konverterleuchtstoff zwischen dem LED-Chip (315, 415) und dem distalen Ende (7) des stabförmigen oder röhrenförmigen Körpers oder am distalen Ende des stabförmigen oder röhrenförmigen Körpers (5) angeordnet ist, dessen Konvertereigenschaften hinsichtlich des von dem LED-Chip (315, 415) emittierten Lichts derart ausgewählt ist, dass er das von dem LED-Chip (315, 415) emittierte Licht in Licht mit einer gewünschten Wellenlängenverteilung konvertiert,
- der Zuleitungsabschnitt (12) an seinem dem distalen hohlen Abschnitt (9) zugewandten Ende eine Kontaktanordnung mit einem ersten Kontakt (323, 423) und einem zweiten Kontakt (342, 425) für den LED-Chip (315, 415) aufweist, **dadurch gekennzeichnet, dass**
- in Bezug auf den Zuleitungsabschnitt (12) der erste Kontakt (323, 423) ein Mittelkontakt (323, 423) und der zweite Kontakt ein Randkontakt (342, 425) ist, wobei der Randkontakt den Mittelkontakt ringförmig umgibt,
- der LED-Chip (315, 415) auf dem Mittelkontakt (323, 423) diesen elektrisch kontaktierend angeordnet ist und mit dem Randkontakt (342, 425) durch einen Bonddraht (341, 441) verbunden ist, und
- der stabförmige oder röhrenförmige Körper (5) einen Außendurchmesser von nicht mehr als 1 mm aufweist.

2. Endoilluminator (1) nach Anspruch 1, **dadurch gekennzeichnet, dass** der Konverterleuchtstoff den LED-Chip (315) in Form eines Vergusses (343) umgibt.

3. Endoilluminator (1) nach Anspruch 1, **dadurch gekennzeichnet, dass** der stabförmige oder röhrenförmige Körper (5) an seinem distalen Ende (7) mit einer für die gewünschte Wellenlängenverteilung durchlässigen Scheibe (445) verschlossen ist und der Konverterleuchtstoff auf die Scheibe (445) aufgebracht oder in die Scheibe (445) eingebracht ist.

4. Endoilluminator (1) nach einem der vorangehenden Ansprüche, **dadurch gekennzeichnet, dass** der Randkontakt (342) in Richtung auf das distale Ende (7) des stabförmigen oder röhrenförmigen Körpers (5) über den Mittelkontakt (323) vorsteht und der Randkontakt (342) den Mittelkontakt (323) spaltfrei umgibt, wobei zwischen dem Randkontakt (342) und dem Mittelkontakt (323) eine elektrische Isolierung (421) vorhanden ist.

5. Endoilluminator (1) nach einem der vorangehenden Ansprüche, **dadurch gekennzeichnet, dass** der distale hohle Abschnitt (9) von einer zylinderförmigen Hülse (347, 447) gebildet ist, in die der LED-Chip (315, 415) eingebracht ist und die auf den Zuleitungsabschnitt (12) aufgesetzt ist.

6. Endoilluminator (1) nach Anspruch 5, **dadurch gekennzeichnet, dass** die Kontaktanordnung einen Sockel bildet, auf den die Hülse (347, 447) aufgesetzt ist.

7. Endoilluminator (1) nach Anspruch 6, **dadurch gekennzeichnet, dass** der Endoilluminator einen Zuleitungsabschnitt (412) aufweist, der als Stäbchen ausgebildet ist und eine zylindrische Hülse (413) sowie ein im Zentrum der zylindrischen Hülse (413) angeordnetes zentrales Stäbchen (417) umfasst, wobei die zylindrische Hülse (413) am distalen Ende des Zuleitungsabschnitts (412) einen Abschnitt (419) aufweist, in dem sie einen reduzierten Außendurchmesser besitzt, und wobei eine distale Hülse (447) auf den Abschnitt (419) mit reduzierten Außendurchmesser aufgesetzt ist, der Innendurchmesser der distalen Hülse (447) dem Außendurchmesser des Abschnitts (419) der zylindrischen Hülse (413) entspricht und der Außendurchmesser der distalen Hülse (447) mit dem Außendurchmesser der Hülse (413) übereinstimmt.

## Claims

1. Endoilluminator (1) comprising:
- an LED chip (315, 415) and
- a rod-shaped or tube-shaped body (5) comprising a proximal end (11), a distal end (7), at least one distal hollow portion (9), which starts in front of the distal end (7) and reaches up to the distal end (7), and a supply portion (12), which starts at the proximal end (11) and reaches up to the distal hollow portion (9),
wherein
- the LED chip (315, 415) is arranged in the distal hollow portion of the rod-shaped or tube-shaped body (5) and supplied with power by way of the supply portion (12),
- a converter phosphor is arranged between the LED chip (315, 415) and the distal end (7) of the rod-shaped or tube-shaped body, or at the distal end of the rod-shaped or tube-shaped body (5), the converter properties of which converter phosphor being selected in respect of the light emitted by the LED chip (315, 415) in such a way that it converts the light emitted by the LED chip (315, 415) into light with a desired wavelength distribution,
- the supply portion (12) has, at the end thereof facing the distal hollow portion (9), a contact arrangement comprising a first contact (323, 423) and a second contact (342, 425) for the LED chip (315, 415),
**characterized in that,**
- in relation to the supply portion (12), the first contact (323, 423) is a central contact (323, 423) and the second contact is an edge contact (342, 425), wherein the edge contact surrounds the central contact in a ring-shaped manner,
- the LED chip (315, 415) is arranged on the central contact (323, 423) in an electrically contacting manner and connected to the edge contact (342, 425) by way of a bond wire (341, 441), and
- the rod-shaped or tube-shaped body (5) has an external diameter of no more than 1 mm.

2. Endoilluminator (1) according to Claim 1, **characterized in that** the converter phosphor surrounds the LED chip (315) in the form of potting (343).

3. Endoilluminator (1) according to Claim 1, **characterized in that** the rod-shaped or tube-shaped body (5) is sealed at the distal end (7) thereof with a pane (445) transmissive to the desired wavelength distribution and the converter phosphor is applied onto the pane (445) or introduced into the pane (445).

4. Endoilluminator (1) according to one of the preceding claims, **characterized in that** the edge contact (342) protrudes over the central contact (323) in the direction of the distal end (7) of the rod-shaped or tube-shaped body (5) and the edge contact (342) surrounds the central contact (323) without a gap, with electrical insulation (421) being present between the edge contact (342) and the central contact (323).

5. Endoilluminator (1) according to one of the preceding claims, **characterized in that** the distal hollow portion (9) is formed by a cylindrical sleeve (347, 447), into which the LED chip (315, 415) is introduced and which is placed onto the supply portion (12).

6. Endoilluminator (1) according to Claim 5, **characterized in that** the contact arrangement forms a base, onto which the sleeve (347, 447) is placed.

7. Endoilluminator (1) according to Claim 6, **characterized in that** the endoilluminator has a supply portion (412), which is embodied as a rod and comprises a cylindrical sleeve (413) and a central rod (417) arranged in the centre of the cylindrical sleeve (413), wherein the cylindrical sleeve (413), at the distal end of the supply portion (412), has a portion (419) in which it has a reduced external diameter, and wherein a distal sleeve (447) is placed onto the portion (419) with a reduced external diameter, the internal diameter of the distal sleeve (447) corresponds to the external diameter of the portion (419) of the cylindrical sleeve (413) and the external diameter the distal sleeve (447) matches the external diameter of the sleeve (413).

## Revendications

1. Illuminateur endoscopique (1), comprenant :
- une puce de LED (315, 415) et
- un corps (5) en forme de tige ou en forme de tube muni d'une extrémité proximale (11), d'une extrémité distale (7), d'au moins une portion creuse distale (9) qui commence avant l'extrémité distale (7) et s'étend jusqu'à l'extrémité distale (7), et une portion de ligne d'arrivée (12) qui commence à l'extrémité proximale (11) et qui s'étend jusqu'à la portion creuse distale (9),
- la puce de LED (315, 415) étant disposée dans la portion creuse distale du corps (5) en forme de tige ou en forme de tube et étant alimentée en énergie par le biais de la portion de ligne d'arrivée (12),
- un luminophore de conversion étant disposé entre la puce de LED (315, 415) et l'extrémité distale (7) du corps en forme de tige ou en forme de tube, ou à l'extrémité distale du corps (5) en forme de tige ou en forme de tube, dont les propriétés de conversion concernant la lumière émise par la puce de LED (315, 415) sont choisies de telle sorte qu'il convertit la lumière émise par la puce de LED (315, 415) avec une distribution de longueurs d'onde souhaitée,
- la portion de ligne d'arrivée (12) possédant à son extrémité qui fait face à la portion creuse distale (9) un arrangement de contact muni d'un premier contact (323, 423) et d'un deuxième contact (342, 425) pour la puce de LED (315, 415), **caractérisé en ce que**
- en référence à la portion de ligne d'arrivée (12), le premier contact (323, 423) est un contact central (323, 423) et le deuxième contact est un contact de bord (342, 425), le contact de bord entourant le contact central en forme d'anneau,
- la puce de LED (315, 415) est disposée sur le contact central (323, 423) en établissant un contact électrique avec celui-ci et est reliée au contact de bord (342, 425) par le biais d'un fil de connexion (341, 441), et
- le corps (5) en forme de tige ou en forme de tube présente un diamètre extérieur inférieur ou égal à 1 mm.

2. Illuminateur endoscopique (1) selon la revendication 1, **caractérisé en ce que** le luminophore de conversion entoure la puce de LED (315) sous la forme d'un scellement (343).

3. Illuminateur endoscopique (1) selon la revendication 1, **caractérisé en ce que** le corps (5) en forme de tige ou en forme de tube est fermé à son extrémité distale (7) avec une plaque (445) transparente pour la distribution de longueurs d'onde souhaitée et le luminophore de conversion est appliqué sur la plaque (445) ou incorporé dans la plaque (445).

4. Illuminateur endoscopique (1) selon l'une des revendications précédentes, **caractérisé en ce que** le contact de bord (342) fait saillie au-dessus du contact central (323) en direction de l'extrémité distale (7) du corps (5) en forme de tige ou en forme de tube et le contact de bord (342) entoure le contact central (323) sans interstice, une isolation électrique (421) étant présente entre le contact de bord (342) et le contact central (323).

5. Illuminateur endoscopique (1) selon l'une des revendications précédentes, **caractérisé en ce que** la portion creuse distale (9) est formée par une douille (347, 447) de forme cylindrique dans laquelle est logée la puce de LED (315, 415) et qui est posée sur ligne d'arrivée (12).

6. Illuminateur endoscopique (1) selon la revendication 5, **caractérisé en ce que** l'arrangement de contact forme un socle sur lequel est posée la douille (347, 447).

7. Illuminateur endoscopique (1) selon la revendication 6, **caractérisé en ce que** l'illuminateur endoscopique possède une portion de ligne d'arrivée (412) qui est réalisée sous la forme d'une baguette et comprend une douille cylindrique (413) ainsi qu'une baguette (417) centrale disposée dans le centre de la douille cylindrique (413), la douille cylindrique (413) possédant à l'extrémité distale de la portion de ligne d'arrivée (412) une portion (419) dans laquelle elle possède un diamètre extérieur réduit, et une douille distale (447) étant posée sur la portion (419) au diamètre extérieur réduit, le diamètre intérieur de la douille distale (447) correspondant au diamètre extérieur de la portion (419) de la douille cylindrique (413) et le diamètre extérieur de la douille distale (447) coïncidant avec le diamètre extérieur de la douille (413).
